# EUROPEAN PATENT APPLICATION

(11) **EP 1 826 274 A1**
(43) Date of publication of application: **29.08.2007**
(21) Application number: 07250780.9
(22) Date of filing: 23.02.2007
(51) Int. Cl.: C12P 19/04, C12P 19/14, C12N 9/26, C12N 1/14

(54) **Enzyme composition, low molecular weight hyaluronan and process for preparing the same**

(30) Priority: 24.02.2006 JP 2006047768
(71) Applicant: KIKKOMAN CORPORATION, Noda-shi, Chiba 278-8601 (JP)
(72) Inventor: Kamei, Jun-ichi, Noda-shi Chiba 278-8601 (JP)
(74) Representative: Denison, Christopher Marcus

(57) **Abstract**

The present invention is to provide a low molecular weight hyaluronan by acting a hyaluronidase derived from a microorganism belonging to the genus *Penicillium* on a hyaluronan and a process for preparing the same, and an enzyme composition which can be suitably used for the preparation of the low molecular weight hyaluronan.

## Description

### [Technical field]

The present invention relates to an enzyme composition, a low molecular weight hyaluronnan and a process for preparing the same.

### [Background art]

Hyaluronnan (in the present specification, the term "hyaluronan" is used in the same meaning as "hyaluronic acid") is a kind of an acidic muco polysaccharide (glycosaminoglycane), and has now been used widely in the fields of medicaments, foods, drinks, cosmetics, and for beauty. It is useful for heightening absorbability of hyaluronan to use a low molecular weight hyaluronan. As a method to make it low molecular weight, it has been well known that hydrolysis by boiling (see Patent Literature 1), a method of using an enzymatic treatment and high-temperature and high-pressure treatment in combination (see Patent Literature 2), etc. Incidentally, as a hyaluronidase which is an enzyme to decompose hyaluronan, in addition to that originated from bovine testes (see Non-Patent Literature 1), those originated from microorganisms belonging to the genus *Streptomyces,* the genus *Propionibacterium,* the genus *Staphylococcus,* the genus *Peptostreptococcus,* and the genus *Streptococcus* (see Non-Patent Literature 2).

[Patent Literature 1] Japanese Unexamined Patent Publication Hei.3-35774
[Patent Literature 2] Japanese Unexamined Patent Publication 2000-102362
[Non-Patent Literature 1] Meyer, K., Hoffman, P., Linker, A. The Enzymes 2nd Ed., (1960) 4, 447
[Non-Patent Literature 2] Park et al. 1997, Biochim. Biophys. Acta, 1337, 217-226

### [Disclosure of the invention]

### [Problems to be solved by the invention]

A preferred aim of the present invention is to provide a low molecular weight hyaluronan and a process for preparing the same. Another preferred aim of the present invention is to provide an enzyme composition which can be used suitably for the preparation of the low molecular weight hyaluronan.

### [Means to solve the problems]

The present inventors have found that hyaluronidase derived from a microorganism belonging to the genus *Penicillium* is suitable for the preparation of a low molecular weight hyaluronan whereby they have accomplished the present invention.
That is, the present invention relates to the following.
(1) A low molecular weight hyaluronan obtained by acting hyaluronidase derived from a microorganism belonging to the genus *Penicillium* on hyaluronan.
(2) A process for preparing a low molecular weight hyaluronan which comprises acting hyaluronidase derived from a microorganism belonging to the genus *Penicillium* on hyaluronan.
(3) A hyaluronidase-containing enzyme composition obtained by culturing a microorganism belonging to the genus *Penicillium* in a culture medium, and collecting an enzyme composition containing hyaluronidase from culture broth.
(4) A process for preparing a hyaluronidase-containing enzyme composition which comprises culturing a microorganism belonging to the genus *Penicillium* in a culture medium, and collecting an enzyme composition containing hyaluronidase from culture broth.

### [Effects of the invention]

According to the present invention, a low molecular weight hyaluronan can be prepared with good efficiency.

### [Brief description of the drawings]

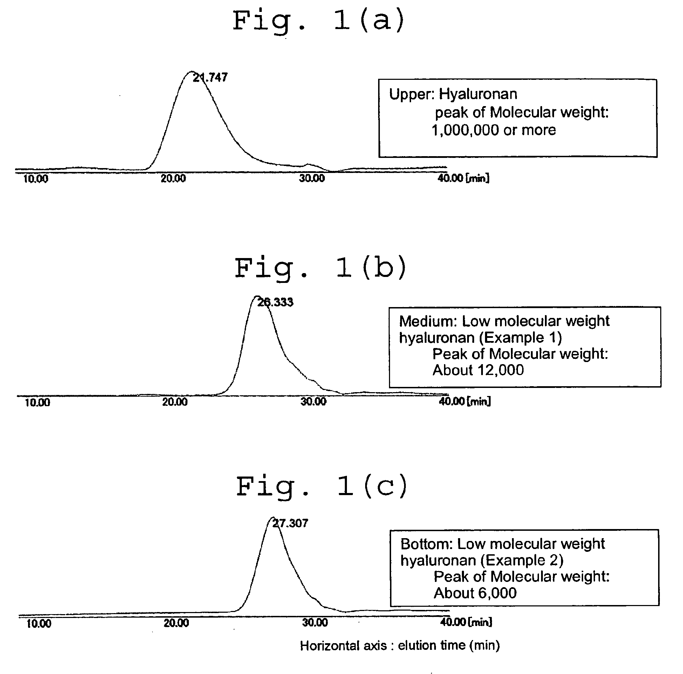

Fig. 1 shows a molecular weight distribution curve according to the gel filtration method of a low molecular weight hyaluronan obtained by using a hyaluronidase-containing enzyme composition originated from *Penicillium Purpurogenum* (Examples 1 and 2).

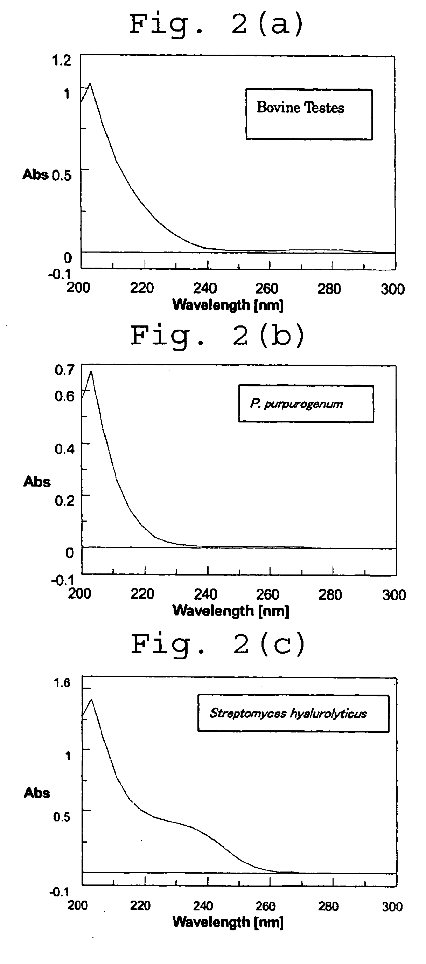

Fig. 2 shows absorption spectra of a low molecular weight hyaluronan obtained by acting a hyaluronidase-containing enzyme composition originated from *Streptomyces hyalurolyticus* (lyase type), originated from bovine testes (hydrolysis type) or originated from *Penicillium purpurogenum* separating with a gel filtration column, and measured at an elution time (10 min) which corresponds to a molecular weight of about 10,000. Fig. 2(a): originated from bovine testes , Fig. 2(b): originated from *Penicillium purpurogenum,* and Fig. 2(c): originated from *Streptomyces hyalurolyticus.*

### [Best mode to carry out the invention]

### [Low molecular weight hyaluronan and process for preparing the same]

The low molecular weight hyaluronan of the present invention can be prepared by acting hyaluronidase or hyaluronidase-containing enzyme composition derived from a microorganism belonging to the genus *Penicillium* on hyaluronan.
As the hyaluronan to be used, its origin or an average molecular weight is not specifically limited, and those obtained from various kinds of animals (a cockscomb, etc.) or microorganisms (*Streptococcus zooepidemicus, Streptococcus equismilis,* etc.) can be used. Also, as the hyaluronan, commercially available products ("hyaluronic acid FCH" available from KIBUN FOOD CHEMIFA CO., LTD., "HYALURONSAN HA-F" available from Q.P. CORPORATION, etc.) may be also used.

The hyaluronidase of the present invention is originated from a microorganism belonging to the genus *Penicillium.* The microorganism belonging to the genus *Penicillium* means, for example, *Penicillium purpurogenum* or *Penicillium funiculosum,* more specifically *Penicillium purpurogenum* (IAM 13753), *Penicillium purpurogenum* (IAM 13754), *Penicillium purogenum* (IAM 7095), and *Penicillium funiculosum* (IAM13752). The hyaluronidase can be prepared by culturing a microorganism belonging to the genus *Penicillium* in a culture medium, and collecting hyaluronidase from the culture broth as an enzyme composition.

The conditions to act the hyaluronidase or hyaluronidase-containing enzyme composition on the hyaluronan may be optionally set depending on the hyaluronan and a kind of the hyaluronidase to be used. For example, when a hyaluronidase-containing enzyme composition originated from *Penicillium purpurogenum* acting on a hyaluronan originated from a microorganism and having an average molecular weight of 1,800,000 to 2,200,000, the enzyme composition is added to a 0.1% hyaluronan solution, and reacted at 4 to 50°C, preferably at 35 to 45°C, for 1 minute to 30 days, preferably about 1 to 48 hours.
Whether a low molecular weight hyaluronan is formed or not according to the above-mentioned reaction can be confirmed by a gel filtration method or a limiting viscosity method. When a molecular weight distribution curve measured by the gel filtration method has a peak caused by the low molecular weight hyaluronan, it can be said that a low molecular weight hyaluronan is formed. Also, when a limiting viscosity method is used for measureing the molecular weight of the product, an average molecular weight of the hyaluronan is measured before and after the treatment with the enzyme composition from a viscosity thereof. When the average molecular weight of the hyaluronan after the treatment with the enzyme composition is small or little, it can be said that a low molecular weight hyaluronan is formed.
The molecular weight of the hyaluronan can be controlled by changing reaction conditions such as a ratio of the hyaluronan and the hyaluronidase, a reaction temperature, a reaction time, etc. By changing reaction conditions, a low molecular weight hyaluronan having a peak of molecular weight on the range of about 400 to about 300,000, preferably in the range of about 400 to about 30,000, more preferably about 5,000 to about 15,000, most preferably about 6,000 to about 12,000, in a molecular weight distribution curve measured by the gel filtration method can be obtained.

### [Hyaluronidase-containing enzyme composition and a process for preparing the same]

The hyaluronidase-containing enzyme composition of the present invention can be prepared by culturing a microorganism belonging to the genus *Penicillium* in a culture medium, and collecting an enzyme composition containing hyaluronidase from a culture broth.
The microorganism belonging to the genus *Penicillium* means, for example, *Penicillium purpurogenum* or *Penicillium funiculosum,* more specifically *Penicillium purpurogenum* (IAM 13753), *Penicillium purpurogenum* (IAM 13754), *Penicillium purpurogenum* (IAM 7095), *Penicillium funiculosum* (IAM13752), etc. The microorganism belonging to the genus *Penicillium* can be obtained from, for example, Institute of Molecular and Cellular Biosciences, The University of Tokyo, Center of Bioinformatics, IAM Culture Collection [IAM Culture Collection (abbreviation: IAM)].
A culturing method of microorganisms and a collecting method of an enzyme composition are not specifically limited as long as a hyaluronidase-containing enzyme composition can be obtained, and, for example, the following methods can be applied. *Penicillium purpurogenum* is planted to a suitable culture medium, and cultured at 25 to 35°C for 3 to 7 days by allowing to stand. As the culture medium, there may be used, for example, a synthetic culture medium, a medium containing starch, a steamed and boiled product of grains such as rice, etc. The obtained culture broth is extracted with a 0.2M NaCl aqueous solution, then, a hyaluronidase-containing enzyme composition can be collected as an extract. Moreover, a content of the hyaluronidase in the hyaluronidase-containing enzyme composition can be heightened by using a usual enzyme-purification method. The thus obtained hyaluronidase-containing enzyme composition can be used for preparing the low molecular weight hyaluronan of the present invention.

### [Examples]

### Example 1

According to the following experiment, it has been confirmed that the hyaluronidase enzyme composition originated from a microorganism belonging to the genus *Penicillium* can be used for the preparation of a low molecular weight hyaluronan.

### 1. Tested strains (4 kinds)

(1) *Penicillium purpurogenum* (IAM 13753)
(2) *Penicillium purpurogenum* (IAM 13754)
(3) *Penicillium purpurogenum* (IAM 7095)
(4) *Penicillium funiculosum* (IAM13752)

### 2. Preparation of hyaluronan

A hyaluronan having an average molecular weigh of about 1,800,000 to about 2,200,000 ("hyaluronic acid FCH FCH200" available from KIBUN FOOD CHEMIFA CO., LTD.) originated from *Streptococcus zooepidemicus* was dissolved in distilled water with a concentration of 0.1% to prepare an aqueous hyaluronan solution.

### 3. Preparation of a hyaluronidase-containing enzyme composition

Test strains were planted to rice medium, and cultured at 30°C for 3 days by allowing to stand. To the cultured product was added an aqueous 0.2M NaCl solution, the mixture was shaken for 1 hour, and then a culture supernatant was prepared by ultracentrifugation of the mixture to obtain a hyaluronidase-containing enzyme composition. As a control, the rice medium was cultured at 30°C for 3 days by allowing to stand, an aqueous 0.2M NaCl solution was added to the medium, and the mixture was shaken for 1 hour and a culture supernatant was prepared by ultracentrifugation.

### 4. Low-molecularization treatment of hyaluronan

To an aqueous hyaluronan solution was added a hyaluronidase-containing enzyme composition with an optional ratio to mix the both, then, the mixture was allowed to stand at 37°C for about 24 hours whereby a low-molecularization treatment of the hyaluronan was carried out.

### 5. Analysis of low molecular weight hyaluronan by gel filtration method

A molecular weight of the hyaluronan contained in a sample after the low-molecularization treatment was analyzed by using a gel filtration column. Conditions of the gel filtration are as follows.
Column: G4000SWXL (available from TOSOH CORPORATION, 7.8x300mm)
   - Eluent: 0.2M NaCl 1 ml/min.
   - Detection: Absorbance (210 nm)
   - Molecular weight standard substance: "Shodex STANDARD P-82" available from SHOWA DENKO K.K. (compound name: pullulan, range of molecular weight: 5,000-800,000)

### 6. Analytical result of low molecular weight hyaluronan

With regard to all four kinds of test strains, molecular weight distribution curves measured by gel filtration method were analyzed, then, it could be confirmed that a peak of the hyaluronan in the molecular weight distribution curve contained in a sample after the low molecularization treatment is confirmed to shift to a low molecular weight side than the peak before the treatment. Also, when an amount of the enzyme composition to be added is increased, or when a time for the treatment is elongated, it could be confirmed that a peak of the molecular weight distribution curve was shifted to a low molecular weight side than that before the treatment. According to the above, it could be confirmed that a low molecular weight hyaluronan had been formed.

In Fig. 1, a molecular weight distribution curve according to the gel filtration method of a low molecular weight hyaluronan obtained by using a hyaluronidase-containing enzyme composition originated from *Penicillium purpurogenum* is shown. A peak of molecular weight in molecular weight distribution curve of the hyaluronan before the low molecularization treatment was 1,000,000 or more (Fig. 1(a)). Also, a peak (elution time: 26.333 min) of the molecular weight in the molecular weight distribution curve of the hyaluronan after subjected to low-molecularization treatment at 37°C for 24 hours was about 12,000 (in Fig. 1(b)).
An elution time of the low molecular weight hyaluronan according to the gel filtration method shown in Fig. 1(b) is 24.5 to 30 minutes. According to the result of gel filtration obtained by using a Molecular weight standard substance (pullulan, "Shodex STANDARD P-82", available from SHOWA DENKO K.K.), the elution time mentioned above correspond to a molecular weight of about 900 to about 47,000.
On the other hand, with regard to the supernatant by ultracentrifugation prepared by the above-mentioned paragraph 3 as a control, there was no change in peaks of the molecular weights distribution curve of the hyaluronan before and after the low-molecularization treatment.

From the above results, all 4 kinds of the tested strains had hyaluronidase-productivity, and gave a hyaluronidase-containing enzyme composition from the cultured product, and it had been shown that the enzyme composition could be used for the preparation of a low molecular weight hyaluronan.

### 7. Analysis of absorbance of low molecular weight hyaluronan

For the purpose of analyzing the low molecularization reaction using the hyaluronidase-containing enzyme composition originated from *Penicillium purpurogenum,* analysis of the absorbance of the low molecular weight hyaluronan was carried out according to the following method.
By using enzyme compositions containing hyaluronidase (lyase type) originated from the genus *Streptomyces,* hyaluronidase (hydrolysis type) originated from bovine testes, and hyaluronidase originated from *Penicillium purpurogenum*, low-molecularization treatment of the hyaluronan was carried out in the same manner as mentioned above. Then, the materials were separated by gel filtration column, and absorbance spectrum was obtained by using a multi-wavelengths detector (MD2010 plus manufactured by JASCO Corporation) with an elution time corresponding to the molecular weight of about 10,000. The results are shown in Fig. 2.
According to comparison of absorbance patterns, it has been suggested that low molecularization reaction using the hyaluronidase-containing enzyme composition originated from *Penicillium purpurogenum* is not a lyase reaction but a hydrolysis reaction.

### Example 2

By using different reaction conditions from those of Example 1, a low molecular weight hyaluronan having a peak at a molecular weight of about 6,000 in a molecular weight distribution curve by the gel filtration method was obtained.
First, in the same manner as mentioned at the paragraphs 2 and 3 of Example 1, an aqueous 0.1% hyaluronan solution and a hyaluronidase-containing enzyme composition were prepared. To the aqueous hyaluronan solution was added the hyaluronidase-containing enzyme composition, and the mixture was allowed to stand at 37°C for 48 hours to carry out the low-molecularization treatment of the hyaluronan.
The mixture after the low-molecularization treatment was analyzed by the same gel filtration method mentioned at the paragraph 5 of Example 1. It has been confirmed that a low molecular weight hyaluronan having a peak (elution time: 27.307 min) at a molecular weight of about 6,000 in a molecular weight distribution curve by the gel filtration method was formed (Fig. 1(c)). An elution time of the low molecular weight hyaluronan shown in Fig. 1(c) obtained by the gel filtration method was 25.5 to 30 minutes. According to the result of the gel filtration obtained by using a Molecular weight standard substance (pullulan, "Shodex STANDARD P-82"), the above elution time corresponds to a molecular weight of about 900 to about 23,000.

### [Utilizability in industry]

According to the present invention, a low molecular weight hyaluronan useful in the fields of medicaments, foods, drinks and cosmetics can be obtained easily and simply.

## Claims

1. A low molecular weight hyaluronan obtained by acting hyaluronidase derived from a microorganism belonging to genus *Penicillium* on a hyaluronan.

2. The low molecular weight hyaluronan according to Claim 1, wherein the obtained hyaluronan has a peak of a molecular weight in the range of about 400 to about 300,000 in a molecular weight distribution curve measured by a gel filtration method.

3. The low molecular weight hyaluronan according to Claim 1, wherein the obtained hyaluronan has a peak of a molecular weight in the range of about 5,000 to about 15,000 in a molecular weight distribution curve measured by a gel filtration method.

4. The low molecular weight hyaluronan according to any one of Claims 1 to 3, wherein the microorganism belonging to genus *Penicillium* is *Penicillium purpurogenum* or *Penicillium funiculosum.*

5. A process for preparing a low molecular weight hyaluronan which comprises acting a hyaluronidase derived from a microorganism belonging to the genus *Penicillium* on a hyaluronan.

6. The process for preparing a low molecular weight hyaluronan according to Claim 5, wherein the resulting low molecular weight hyaluronan has a peak of a molecular weight in the range of about 400 to about 300,000 in a molecular weight distribution curve measured by a gel filtration method.

7. The process for preparing a low molecular weight hyaluronan according to Claim 5, wherein the resulting low molecular weight hyaluronan has a peak of a molecular weight in the range of about 5,000 to about 15,000 in a molecular weight distribution curve measured by a gel filtration method.

8. The process for preparing a low molecular weight hyaluronan according to any one of Claims 5 to 7, wherein the microorganism belonging to genus *Penicillium* is *Penicillium purpurogenum* or *Penicillium funiculosum.*

9. A hyaluronidase-containing enzyme composition obtained by culturing a microorganism belonging to the genus *Penicillium* in a culture medium, and collecting an enzyme composition containing hyaluronidase from a culture broth.

10. The hyaluronidase-containing enzyme composition according to Claim 9, wherein the microorganism belonging to genus *Penicillium* is *Penicillium purpurogenum* or *Penicillium funiculosum.*

11. A process for preparing a hyaluronidase-containing enzyme composition which comprises culturing a microorganism belonging to the genus *Penicillium* in a culture medium, and collecting an enzyme composition containing hyaluronidase from a culture broth.

12. The process for preparing a hyaluronidase-containing enzyme composition according to Claim 11, wherein the microorganism belonging to genus *Penicillium* is *Penicillium purpurogenum* or *Penicillium funiculosum.*
